# EUROPÄISCHE PATENTSCHRIFT

(11) **EP 1 170 024 B1**
(45) Veröffentlichungstag und Bekanntmachung des Hinweises auf die Patenterteilung: **29.10.2003**
(21) Anmeldenummer: 01102303.3
(22) Anmeldetag: 01.02.2001
(51) Int. Cl.: A61M 5/142

(54) **Vorrichtung zur Injektion eines Medikamentes**
Device for the injection of a medicament
Dispositif pour l'injection d'un médicament

(30) Priorität: 02.02.2000 DE 10004496
(43) Veröffentlichungstag der Anmeldung: 09.01.2002
(73) Patentinhaber: Lamster, Jörg, Dr. med., 99097 Erfurt (DE)
(72) Erfinder: Lamster, Jörg, Dr. med., 99097 Erfurt (DE); Roscher, Dietrich, Dr.-Ing., 99099 Erfurt (DE)
(74) Vertreter: Enders, Hans, Dipl.-Ing.

(56) Entgegenhaltungen:
- WO-A-97/36623
- WO-A-99/32174
- DE-A- 19 848 229
- US-A- 5 860 957

## Beschreibung

Die Erfindung betrifft eine Vorrichtung zur Injektion eines Medikamentes mit der Vorgabe und Änderung einer dosierten Menge , mit einem Behälter, der ein gespeichertes flüssiges Therapeutikum enthält, einer Injektionseinrichtung und einer Fördereinrichtung, insbesondere zur regelmäßigen Injektion von z.B.Insulin, vorwiegend bei Patienten, die zur Selbstinjektion nicht fähig sind.

Zum Stand der Technik gehören sogenannte Insulin-Pens. Dabei handelt es sich um ein Gerät in Form eines Füllfederhalters mit einem kleinem Glaszylinder, der das Medikament enthält. Die eine Stirnfläche des Glaszylinders öffnet sich in eine Injektionskanüle, auf der entgegengesetzten Seite des Glaszylinders ist ein verschiebbarer Stempel, der die Medikamentenflüssigkeit durch der Kanüle austreibt, angeordnet. Ein drehbares Einstellrad an diesem Pen ermöglicht die Einstellung der zu injizierenden Menge. Durch anschließendes manuelles Drücken oder Verschieben eines Bedienknopfes wird über den Stempel die eingestellte Medikamentenmenge durch die Kanüle ausgetrieben. Das System ist relativ einfach zu handhaben, stellt aber insbesondere für körperlich und geistig Behinderte / Demenzkranke eine erhebliche Schwierigkeit dar. Z.B. bedarf die Einstellung der Injektionsmenge sowohl eines durchschnittlichen manuellen Geschickes, als auch der genauen Kenntnis oder eines ärztlichen Planes über die Mengenangabe, der gelesen oder dessen Inhalt gemerkt werden muß. Dabei ist anzumerken, daß sich viele Patienten drei- bis viermal täglich mit unterschiedlichen Mengen behandeln müssen. Weiterhin besteht keine Kontrolle darüber, ob der Wirkstoff überhaupt und vollständig injiziert worden ist. Es weist auch keine optische oder akustische Vorrichtung auf, die den Patienten an eine zu vollziehende Injektion erinnern.

Bekannt sind auch Insulinpumpen, die am Körper von Diabetikern getragen werden können und über eine permanent in der Haut des Patienten liegende Kanüle nach einer definierten Fluß-Zeitfunktion dem Körper Insulin zuführen.
Diese Vorrichtung hat den Nachteil einer dauerhaft im Körper des Patienten verweilenden Kanüle und ist deshalb nur einer kleinen Gruppe von Diabetikern vorbehalten. Zur Regulation der Flußmenge ist jeweils eine erneute Voreinstellung des Gerätes erforderlich und gestattet keinen Eingriff bei Abwesenheit. Eine Rückkopplung über das Befinden des Patienten an den Arzt, sowie ein folgender Eingriff durch den Arzt ist nicht vorhanden.

In der DE 196 43 813 wird eine Vorrichtung mit einer Injektionspumpe bekannt, die stationär verwendet wird und vorwiegend in der Intensivmedizin Verwendung findet, um einen medizinischen Wirkstoff kontinuierlich intravenös zuzuführen. Sie besteht aus einer Kolbenspritze, die über eine Kolbenstange entsprechend einer programmier-baren Vorgabe geleert wird. Der Kolbenvorschub erfolgt dabei geregelt, d.h. es erfolgt ein SOLL-ISTVergleich für die Kolbenverfahrvorrichtung. Ein Regelkreis dient dazu, einen Elektromotor als Antrieb für die Kolbenstange über den SOLL-IST-Vergleich zu steuern, um auch bei unterschiedlichen Spritzengrößen die Vorgabemenge zu injizieren. Der Kolbenvorschub ist also ein Maß für die Injektionsmenge.
Zum ambulanten Gebrauch sind diese Vorrichtungen nicht geeignet. Darüberhinaus sind sie an eine im Körper verweilende Kanüle gebunden. Eine äußere Eingriffsmöglichkeit durch medizinisches Personal besteht nicht.

Weiterhin sind Vorrichtungen in Form von Penholders, sogenannten Druck-Injektomaten bekannt, die keine Injektionskanüle mehr aufweisen, Diese Injektomaten werden manuell auf die zuvor gereinigte Haut aufgesetzt und per Druck wird das Therapeutikum in die Hautfläche eingedrückt. Das Gerät ist einfach in der Handhabung, eine Rückkopplung über
das Befinden des Patienten an den Arzt bei örtlicher Abwesenheit des Patienten ist nicht vorhanden. Das Gerät läßt keine Kontrolle über die Eindringtiefe - bei unterschiedlichem Hautwiderstand - und die tatsächlich realisierte Injektionsmenge in die Haut zu. Eine programmierte Eingabe der zu injizierenden Menge ist nicht vorgesehen. Eine äußere bzw. externe Beeinflussung und Überwachung der Gerätefunktionen durch den Arzt oder medizinisches Personal bei örtlicher Distanz des Patienten sind nicht möglich. Das Gerät ist in seiner Handhabung auch nicht für körperlich und geistig Behinderte / Demenzkranke geeignet.

In der DE 195 36 823 wird eine Prüfvorrichtung beschrieben, mit der eine Dosierpumpe vermessen wird, wobei eine Gegenkraft als Maß der Flußgröße ermittelt wird und als steuerbare Kraftquelle ausgebildet ist. Die Vorrichtung ist für den stationären Bereich vorgesehen und erkennt nicht die tatsächliche in oder unter die Haut injizierte Menge.

In der WO-A-99/32174 wird ein Medikamentenspender beschrieben, der vorgibt, bei einer Injektion einen Flüssigkeitsaustritt oder einen Lufteintritt auszuschließen. Das Medikamentenreservoir wird mit einem Pflaster auf der Haut befestigt und ein Kanülenrohr verbleibt während der Befestigung in der Haut des Patienten, beziehungsweise wird ein Schlauch mittels Einstichhilfe in die Haut eingebracht, der dort verbleibt. Das schränkt die Bewegungsfreiheit des Patienten erheblich ein. Zur Verdrängung des Medikamentes aus dem Reservoir werden die verschiedensten Medien, beispielsweise Edelgase, Luft oder Kochsalzlösung verwendet. Der eingesetzte Sensor mißt den Fluß der aus der Vorrichtung austretenden Flüssigkeit oder eines treibenden Pumpmediums , nicht jedoch die tatsächlich in der Haut eingeführte Menge.

In der US 5 860 957 wird ein Nadelantrieb für eine Injektionsvorrichtung beschrieben, der auf einem Kolben-/Zylinder-Prinzip beruht und bei dem die Nadel mittels Gasdruck in beide Richtungen bewegt wird.

Der Erfindung liegt daher die Aufgabe zugrunde, eine am Körper eines Patienten tragbare Vorrichtung zur wiederholbaren Injektion eines Medikamentes zu schaffen, die eine flüssige Medikamentenmenge selbsttätig in die Haut injiziert und kontrolliert, den sicheren Kontakt der Vorrichtung zur Haut ermittelt, Fehlfunktionen alarmiert und die aseptischen Anforderungen erfüllt. den Patienten zeitlich an die Benutzung des Systems erinnert, die Gerätefunktionen ortsunabhängig überwacht, eine Programmänderung extern zuläßt und jederzeit eine Spachverbindung mit einer externen Überwachungsstelle ermöglicht.

Diese Aufgabe der Erfindung wird durch eine am Körper des Patienten tragbare Injektionseinrichtung nach Anspruch 1 gelöst. Sie verfügt neben einer Dosier- und Fördereinrichtung für ein Therapeutikum über eine Vorrichtung zur Penetration einer Injektionskanüle und über eine aus mehreren Sensoren bestehende Sensorik und wirkt mit einer integrierten programmierbaren Informationsverarbeitungseinheit zusammen, die gespeicherte Daten über die Injektionszeiten und -menge, sowie ein Ablaufprogramm zur Auswertung der Sensorik und zum Ablauf der Injektion in einem internen Regelkreis enthält und einen Übertragungsmodul für die wechselseitige Datenübertragung von Meßwerten, Stellgrößen und separater Sprachverbindung zwischen dem Träger der Vorrichtung und einer externen Zentralstation aufweist, wobei über den Übertragungsmodul eine externe Überwachung der Körperfunktion und aller Gerätefunktionen erfolgt bzw. erfolgen kann, sowie die in der Vorrichtung gespeicherten medizinischen Daten und die Programmierung änderbar sind. Über dieses Übertragungsmodul, z.B. ein GSM (Modem bzw. Mobiltelefon), ist über ein Funknetz, z.B. D I oder D2, eine wechselseitige Sprachverbindung zwischen Patient und externer Zentralstelle vorgesehen und jederzeit möglich.

Die Sensorik für den geräteinternen Regelkreis besteht dabei aus mehreren Auflagesensoren , deren Signale auf eine logische Schaltung geführt sind und die Bereitschaft der Vorrichtung für eine Injektion signalisieren. Weitere Sensorsignale überwachen die Penetration einer Injektionskanüle in die Haut und kontrollieren die Ausführung der injizierten Menge in die Haut. Die Sensorsignale werden von der internen Informationsverarbeitungseinheit für die weitere Programmfolge ausgewertet.
Die Informationsverarbeitungseinheit verfügt dabei über eine Zeit-Mengen-Steuerung z.B. mit einem Register für ZEIT und einem Register für MENGE, und steuert u.a. eine Antriebseinheit für die Injektionskanüle und eine Dosiervorrichtung mit Pumpvorrichtung.

Vorzugsweise mindestens 3 Auflagesensoren sind in der Grundplatte der Vorrichtung, die u.a. auch zur Körperauflage am Patienten dient, angeordnet. Bei korrekter Körperauflage der Vorrichtung wird das Ausgangssignal eines jeden Auflagensensors an ein logisches UND-Gatter geführt und ein Bereitschaftssignal zur Auswertung für die Informationsverarbeitungseinheit gebildet.

Das sichere und korrekte Eindringen der Injektionsnadel in die Haut erfolgt vorzugsweise über eine Impedanzmessung, so daß erst nach Vorliegen eines entsprechenden Kontrollsignales die Injektion mit dem Therapeutikum ausgeführt wird.

Die tatsächliche Injektion in die Haut wird vorzugsweise von einem IR-Sensor kontrolliert und als auswertbares Signal an die Informations-verarbeitungseinheit übertragen.

Die Vorrichtung besteht vorzugsweise in einem kompakten von einem Gehäuse umgebenen Gerät, das im Kern zur Erfüllung der aseptischen Anforderungen an ein solches Gerät aus einem geschlossenem Fluidbereich besteht, an dem die weiteren erforderlichen Baugruppen, wie Antrieb für die Injektionskanüle, Fördervorrichtung für das flüssige Therapeutikum, Stellglieder für Ventile. Sensor- und Meßstellen, ausschließlich außerhalb des Fluidflusses angeordnet sind und mit dem Medium nicht in Berührung kommen. Dieser geschlossene Fluidbereich besteht dazu aus einem strukturiertem Kanalsystem in einem flexiblen Folienverbund, der im wesentlichen aus zwei übereinanderliegenden, miteinander verschweißten oder verklebten Folien, z.B. aus Polytetrafluoräthylen (PTFE), besteht.
Dieses Material ist chemisch resistent und erfüllt die aseptischen Anforderungen.

Die Struktur des Kanalsystems umfaßt vorzugsweise ein Depot für das Therapeutikum, Ventilkammer, Pumpkamnier, Fluidkanal und Einlaß zur Auffüllung des Depots und Auslaß für eine anzuschließende Kanüle. Nach längerem Gebrauch kann dieser Folienverbund leicht ausgewechselt werden.

Eine weitere Baugruppe der Vorrichtung besteht in der Dosiereinrichtung der voreinstellbaren Menge des Medikamentes. Zu dieser Baugruppe gehören vorzugsweise ein Ventil, eine steuerbare Pumpe , eine Flußmeßeinrichtung, die in einem Kanalkörper angeordnet sind. Als steuerbare Pumpe ist vorzugsweise eine ventillose Mikropumpe angeordnet, an deren Pumpkammer ein Piezo-Bimorph-System als Antriebsmembran angeordnet ist und an deren Ein-und Ausgang richtungsabhängige Strömungswiderstände liegen.

Eine weitere wesentliche Baugruppe besteht in einer Antriebsvorrichtung für die am Fluidbereich angeschlossene Injektionsnadel, damit die Injektionsnadel vorzugsweise schräg in einem spitzem Winkel von ca. 45 bis 60° aus der Vorrichtung heraustreten und in die Haut des Patienten eingeführt werden kann.
Diese Antriebsvorrichtung besteht vorzugsweise aus einem mechanischem Linearantrieb unter Verwendung piezoelektrischer Aktorelemente, die in ihrer Anordnung einen Schubverband für die Injcktionsnadel bilden. Die Piezoaktoren bestehen dabei aus zwei Klemmringen mit je einer mittigen Bohrung zur Durchführung der Injektionsnadel und einem zwischen den Klemmringen eingesetzten hülsenförmigem Schubring. Die Stirnflächen des Schubringes sind jeweils mit einer Seitenfläche der Klemmringe verbunden, z.B. verklebt. Bei Anlegen einer Spannung an einen Klemmring zieht sich dessen mittige Bohrung zusammen und bewirkt mit ihrer Klemmfläche eine kraftschlüssige Verbindung mit der Zylinderfläche der durchgeführten Injektionsnadel. Bei Anlegen einer Spannung an den Schubring erfährt dieser eine Längenausdehnung und drückt mit seinen Stirnflächen die beiden Klemmringe auseinander. Werden die Piezoaktoren in entsprechender zeitlicher Folge angesteuert, ergibt sich ein Schubverband für eine lineare Bewegung der Injektionsnadel. Die Füllung des Depots erfolgt über eine Ladestation in der Vorrichtung, in die eine handelsübliche Kartusche eingelegt wird und deren Inhalt über eine Kontaktnadel und 5 durch manuelle Kolbenbewegung in die Depots übertragen wird.

Während die Ausführung der Gerätefunktionen über die Menge-Zeit-Steuerung erfolgt, sich das System über die Sensorik selbst kontrolliert, werden die MENGE und die ZEIT in Abhängigkeit der ermittelten medizinischen Daten über ein GSM (Modem) eines Übertragungsmoduls extern durch Verbindung mit einer Zentralstelle verändert bzw. beeinflußt.
Weiterer Bestandteil des Übertragungsmoduls ist ein Mobil-Telefon-Schaltkreis für eine wechselseitige Sprachverbindung zwischen Patient und externer Zentralstelle. Der Patient wird vorzugsweise außerdem, falls er innerhalb einer vorgeschriebenen Zeit keine Aktivierung vorgenommen hat, optisch und akustisch auf eine durchzuführende Injektion aufmerksam gemacht.

Weitere Einzelheiten werden durch die in den Ansprüchen 1 bis 19 dargelegten Merkmale gelöst.

Der der Erfindung zugrundeliegende Gedanke wird in der nachfolgenden Beschreibung anhand eines Auslührungsbeispieles, das in der Zeichnung näher dargestellt ist, erläutert.

Es zeigen:
- Fig. 1: ein Blockschaltbild für den internen Regelkreis der Vorrichtung und die externe Kommunikation der Vorrichtung über ein Übertragungsmodul mit einer Zentralstelle ;
- Fig. 2: eine Prinzipdarstellung der Vorrichtung mit der Anordnung der funktionellen Baugruppen und deren Verbindungen;
- Fig. 3: eine Prinzipdarstellung der Vorrichtung gern. Fig. 2 mit erkennbarer Darstellung des Medikarnenten-Depots;
- Fig. 4: eine Draufsicht auf das Kanalsystem mit Depots in einem Folienverbund;
- Fig. 4.1: Draufsicht auf das Kanalsystem gern. Fig. 4 mit Ventil für die Kartuschenentnahme;
- Fig. 5: eine Draufsicht auf die Kanalstruktur der Infusionspumpe
- Fig. 5.1: ein Querschnitt der Infusionspumpe gern. Fig. 5;
- Fig. 6: eine Prinzipdarstellung einer piezoelektrischen Antriebs vorrichtung für die Injektionsnadel;
- Fig. 6.1: ein Impulsdiagramm für die Ansteuerung der Antriebsvorrichtung gern. Fig. 6;
- Fig. 7: Querschnitt durch ein Fluidventil gern. Fig. 2 - bei geschlossenem Ventil;
- Fig. 7.1: Querschnitt durch ein Fluidventil gern. Fig. 2 - bei geöffnetem Ventil;
- Fig. 8: eine prinzipielle Anordnung einer Sensorik zur Kontrolle der erfolgten Infusion;
- Fig. 8.1: eine schematische Anordnng der Auflageelektroden, IR-Sensor und Injektionsstelle in der Haut;
- Fig. 8.2: ein logische Schaltungsanordnung zur Auswertung der Auflagesensoren und der Penetration der Injektionsnadel;
- Fig. 9: ein Impulsdiagramm für die Auswertung der Signale gem. Fig. 8.2;

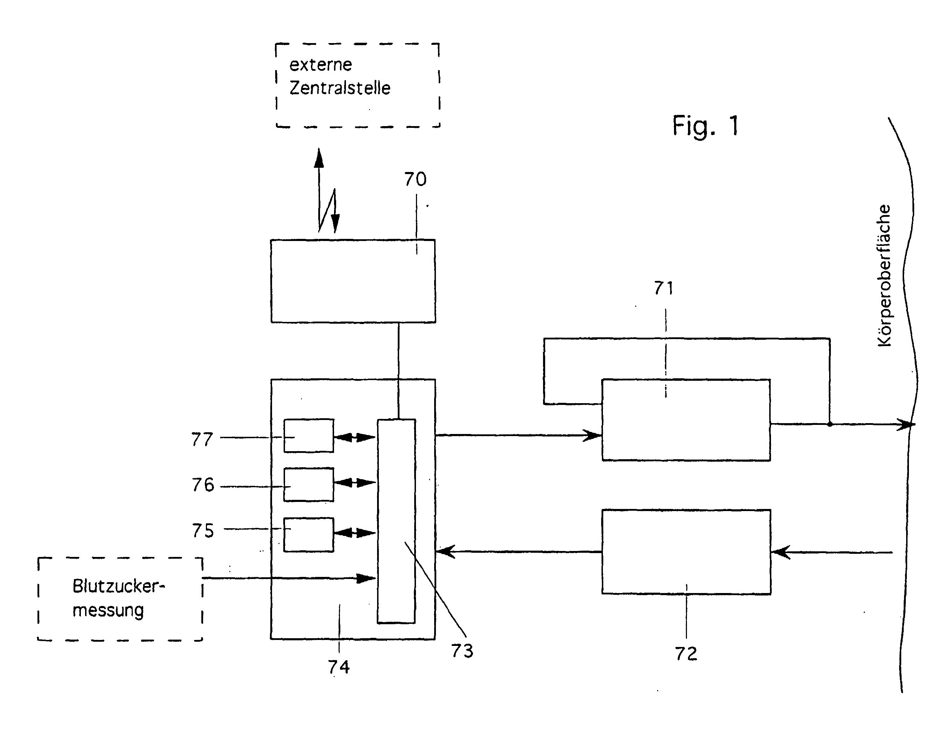

Die Vorrichtung zur Injektion eines Medikamentes dosierter Menge in die Haut eines Patienten besteht darin, daß sie aus einem die Injektion ausführenden System (Fig.1) besteht, das sich durch eine Sensorik in Verbindung mit einer internen Informationsverarbeitungseinheit 74 selbst kontrolliert, wobei in der Informationsverarbeitungseinheit ein Register 77 für die MENGE und ein Register 76 für die ZEITEN gespeichert sind. eine Mengen-Zeit-Steuerung für einen programmierten Injektionsablauf sorgt und über einen Übertragungsmodul 70 eine Datenübertragung an eine externe Zentralstelle 78 und eine Kommunikation der Zentralstelle zwecks Änderung der Registerinhalte und Änderung des Programmablaufs in der Vorrichtung erfolgt und über ein Mobil-Telefon-Schaltkreis eine wechselseitige Sprachverbindung, z.B, über ein D 1-oder D2-Netz, zwischen externer Zentralstelle und Patient erfolgen kann.

Mit der Vorrichtung besteht nicht nur die Möglichkeit einer programmierten und sich selbst kontrollierenden Durchführung einer Injektion, sondern auch eine externe Überwachung eines Patienten durch medizinisches Personal und eine externe Änderung der Programmabläufe und Änderung der Registerinhalte in der am Körper befindlichen Vorrichtung.

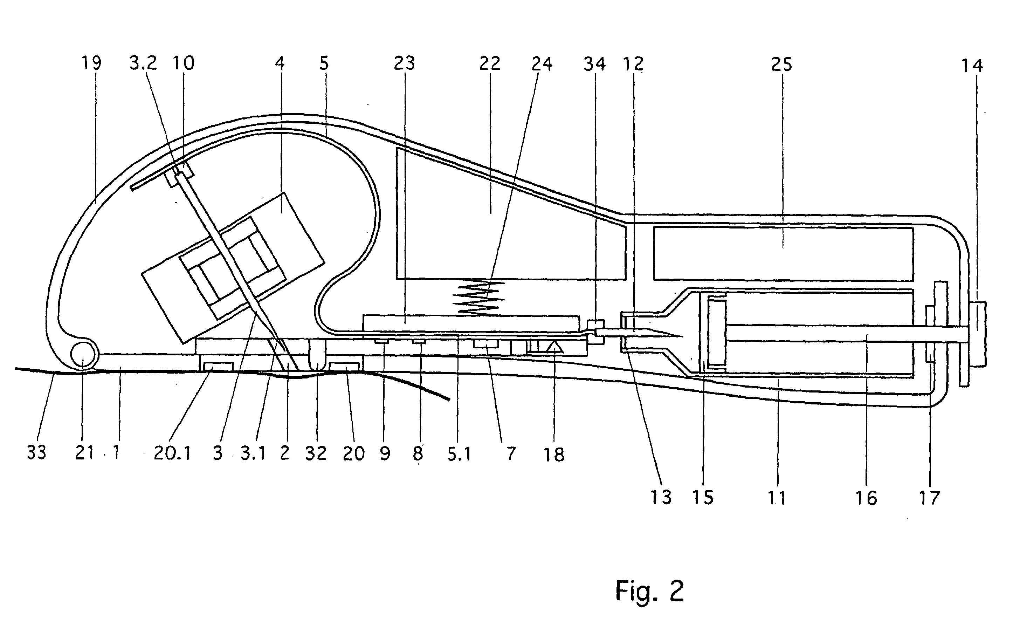

Die Vorrichtung (Fig.2) besteht vorallem aus einer kleinen kompakten und am Körper tragbaren Einheit, die nur einen geringen Energiebedarf hat, über einen geschlossenen Fluidbereich für das Therapeutikum verfügt, alle weiteren Baugruppen, wie Antrieb 4 für die Injektionskanüle 3, Fördervorrichtung 7 für das flüssige Therapeutikum, Stellglieder für Ventile, Sensor- und Meßstellen , nur außerhalb des Fluidflusses angeordnet sind und somit nicht mit dem Therapeutikum in Berührung kommen und damit die aseptischen Anforderungen an ein solches Gerät erfüllen.

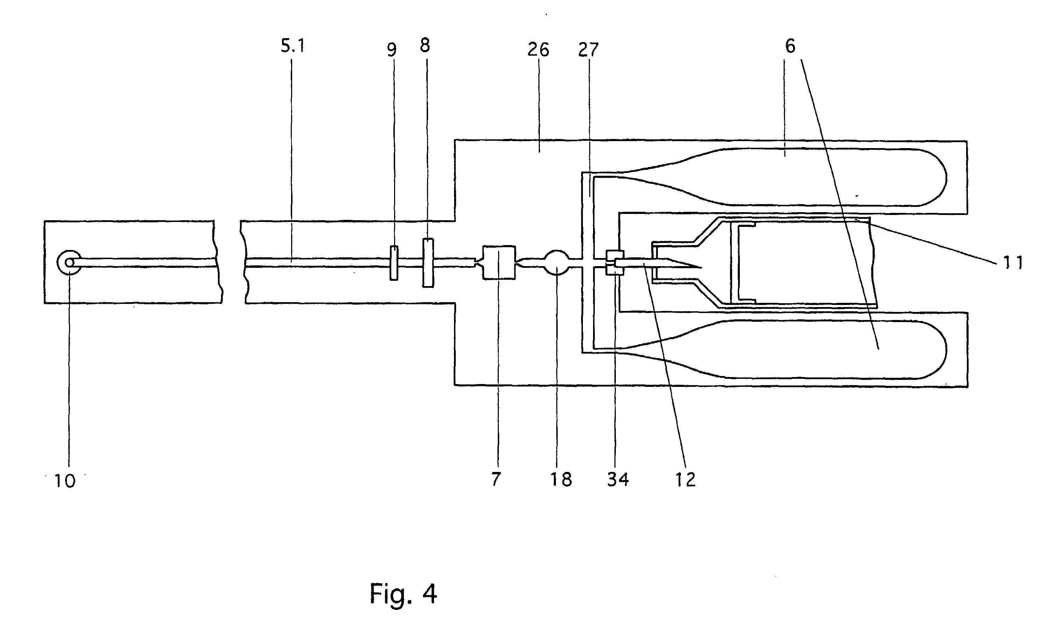

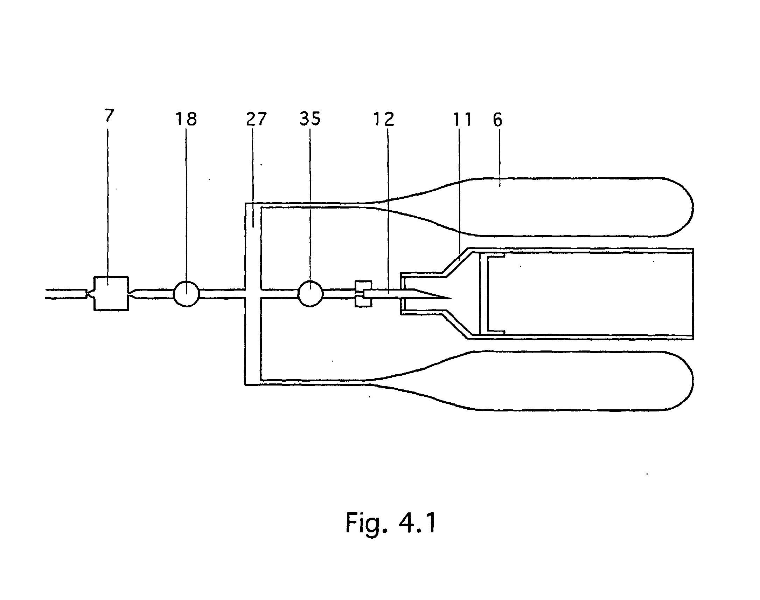

Dieser geschlossene Fluidbereich, in Fig.4; 4.1 näher erkennbar, besteht dazu aus einem strukturiertem Kanalsystem in einem flexiblen Folienverbund 26, der im wesentlichen aus zwei übereinanderliegenden, miteinander verschweißten oder verklebten Folien, z.B. aus Polytetrafluoräthylen (PTFE), besteht und gegen das Therapeutikum chemisch resistent ist.

Die Struktur des Kanalsystems umfaßt ein Depot 6 für das Therapeutikum, eine Ventilkammer 18, eine Pumpkammer 7, einen Fluidkanal 5.1 und Einlaß 34 zur Auffüllung des Depots und Auslaß 10 für eine anzuschließende Injektionskanüle oder -nadel 3. Nach längerem Gebrauch kann dieser Folienverbund 26 leicht ausgewechselt werden.

Dazu soll ein Ausführungsbeispiel für die Vorrichtung näher beschrieben werden. Die Vorrichtung (Fig.2) ist auf einer am Körper des Patienten anliegenden Grundplatte 1 aufgebaut. In der Grundplatte 1 sind mindestens drei , vorteilhaft vier Auflagesensoren 20 eingesetzt, über die eine sichere flächenhafte Auflage der Vorrichtung auf der Körpers oberfläche des Patienten kontrolliert wird. Nur bei sicherer und flacher Körperauflage wird die Vorrichtung in den Status einer Funktionsbereitschaft versetzt. Weiterhin ist in der Grundplatte 1 eine Öffnung 2 eingebracht, durch die eine Injektionsnadel 3 im spitzem Winkel von etwa 45 bis 60° linear heraustreten kann.

Der Grundaufbau der Vorrichtung besteht also aus einer schräg geführten Injektionsnadel 3, die mit einem mechanischen Linearantrieb 4 in Verbindung steht. Das Ende 3.1 der Injektionsnadel 3 ist am Ausgang des Fluidbereiches mit einer Fluidleitung 5 des Folienverbundes 26 verbunden, über die ein Medikament in die Injektionsnadel 3 geleitet wird. Die Fluidleitung 5 führt an ein Depot 6, in dem sich z.B. Insolin oder ein ähnliches Therapeutikum befindet. Zwischen dem Depot 6 und der Fluidleitung 5 befindet sich an einem Fluidkanal 5.1 eine Fördereinrichtung, z.B. eine Pumpe 7, die das Medikament aus dem Depot 6 in die Injektionsnadel 3 fördert. Die Pumpe 7 wird von der Informationsverarbeitungseinheit 74 so gesteuert, daß vorbestimmte in einem Register MENGE gespeicherte oder über ein Modem übermittelte Fluidmengen und zu einer in einem weiteren Register festgelegten ZEIT an den Ausgang 3.1 der Injektionsnadel 3 gefördert werden.

Eine Zeit-Mengcn-Steuerung in der Informationsverarbeitungseinheit sorgt dafür, daß eng tolerierte Fluidmengen am Ausgang 3.1 der Injektionsnadel 3 zur Über tragung in die Haut 33 des Patienten gelangen. Für die Zeit-Mengen- Steuerung sind am Ausgang der Pumpe 7 in definiertem Abstand ein Flußmesser, der in einem Peltierelement 8 und einem Temperatursensor 9 besteht, angeordnet. Über das Peltierelement 8 werden Temperaturänderungen , z.B. eine Erwärmung oder eine Abkühlung des Therapeutikums, vorgenommen, die vom Temperaturelement 9 nach einer Zeit t wahrgenommen werden. Über die konstanten Kanaldimensionen und dem zeitlichen Abstand zwischen HEISS und KALT des Fluids entsteht ein Maß für die Flußgeschwindigkeit und damit für die Durchflußmenge. Die Durchflußmenge, die für die Injektion erforderlich ist, wird somit in einem SOLL-IST-Vergleich über die Pumpe 7 mit hoher Genauigkeit meß- und steuerbar.

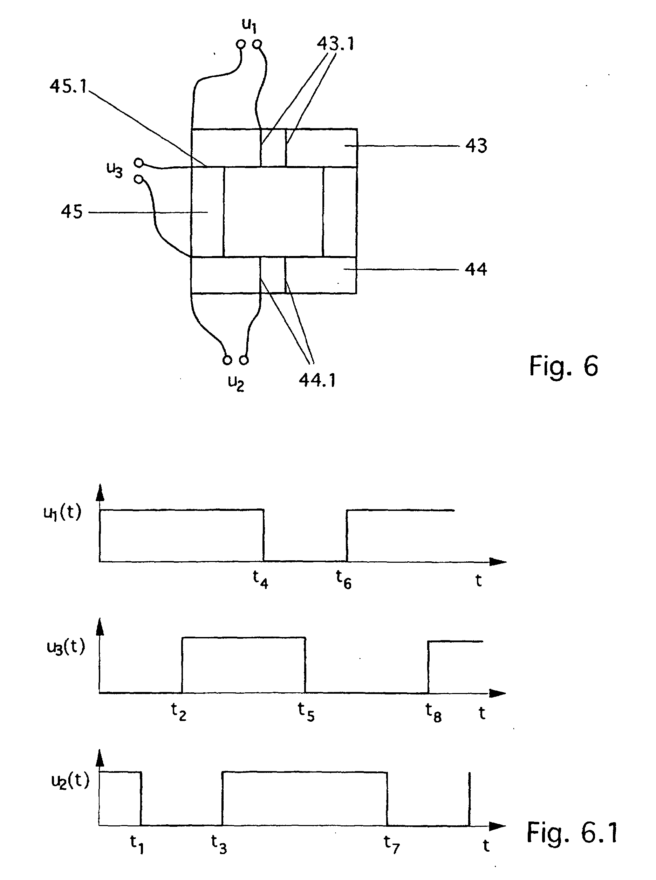

Zuvor wird über einen Sensor bzw. eine Impedanzmessung kontrolliert, ob die Injektionsnadel 3 tatsächlich in die Haut des Patienten eingedrungen ist. Die Kontrolle einer erfolgreichen Injektion geschieht mit Hilfe eines IR-Sensors 32, der in der Bodenplatte 1 integriert, ist und dem Injektionsbereich der Haut 33 gegenübersteht. Die erfolgreiche Injektion bewirkt einen Temperaturabfall der unter dem IR-Sensor 32 liegenden Hautschicht und liefert ein Signal zur Aufzeichnung und für eine optische und akustische Anzeige. Der mechanische Linearantrieb 4, der die Injektionsnadel 3 im spitzem Winkel aus der Grundplatte 1 der Vorrichtung bewegen kann, besteht aus einem Vibrationsantrieb unter Verwendung piezoelektrischen Elemente. Je nach Ansteuerung dieser piezoelektrischen Elemente wird die Injektionsnadel 3 durch die Öffnung 2 in der Gehäusegrundplatte 1 nach außen und mit ihrer Spitze 3.1 in die Haut 33 eingeführt und nach erfolgter Injektion wieder zurückgezogen. Ein Ablaufprogramm in der Informationsverarbeitungseinheit sorgt für die Ansteuerung dieses Antriebes. Die Linearbewegung ist zusätzlich von einem mechanischem Anschlag begrenzt.
Der Linearantrieb 4 (Fig. 6) unter Verwendung piezoelektrischer Aktorelemente bildet in seiner Anordnung einen Schubverband für die Injektionsnadel 3. Die Piezoaktoren bestehen dabei aus zwei Klemmringen 43; 44 mit je einer mittigen Bohrung mit den Klemmflächen 43.1; 44.1 zur Durchführung der Injektionsnadel 3 und einem zwischen den Klemmringen eingesetztem hülsenförmigem Schubring 45. Die Stirnflächen 45.1 des Schubringes 45 sind jeweils mit einer Seitenfläche der Klemmringe 43; 44 verbunden, z.B. verklebt. Bei Anlegen einer Spannung U an einen Klemmring zieht sich dessen mittige Bohrung 43.1; 44.1 zusammen und bewirkt eine kraftschlüssige Verbindung mit der Zylinderfläche der durchgeführten Injektionsnadel 3. Bei Anlegen einer Spannung an den Schubring 45 erfährt dieser eine Längenausdehnung und drückt mit seinen Stirnflächen 45.1 die beiden Klemmringe 43; 44 auseinander. Werden die Piezoaktoren in ent- sprechen der zeitlicher Folge angesteuert, ergibt sich ein Schubverband für eine lineare Bewegung der Injektionsnadel 3. Durch Änderung der Phasenlage der Ansteuersignale erfolgt eine Rückwärtsbewegung der Injektionsnadel 3.

In Fig. 6.1 ist eine mögliche zeitliche Impulsfolge für die Ansteuerung der 3 Piezoaktoren 43; 44; 45 aufgezeigt. Einer der beiden Klemmringe ist dabei mit dem Chassis fest verbunden.

Das Ende des Fluidkanals 5.1 ist mit dem Depot 6 für das Medikament verbunden. Das Depot 6 wird über eine Kartusche 11 gefüllt, die zuvor in die Vorrichtung eingelegt wird. Beim Einlegen der Kartusche 11 dringt eine Kontaktnadel 12 in die Verschlußkappe 13 z.B. aus Naturkautschuk oder Latex , der Kartusche 11 ein und stellt die Fluidverbindung 5 zwischen Depot 6 und der Kartusche 1 1 her. Über einen von Hand betätigten Kolben 15 wir die Kartusche 11 vor dem Anlegen der Vorrichtung an den Körper des Patienten entleert. Das Fluid wird dabei in das Depot 6 übertragen. Ein Ventil 18 sorgt u.a. dafür, daß das Fluid bei der Entleerung der Kartusche 11 nicht in den Fluidkanal 5.1 und durch die Injektionsnadel 3 gedrückt wird. Die Kartusche 11 kann so in der Vorrichtung verbleiben, wobei der Kolben 15 über einen Stempel 16 und Betätigungsknopf 14 mittels Verriegelung 17 arretiert wird. Die Kartusche 11 kann aber auch nach der Entleerung in das Depot 6 wieder entnommen werden, wobei ein weiteres Ventil 35 (Fig. 4.1) für einen sicheren Verschluß des Fluidbereiches sorgt.

Der elektronische Teil der Vorrichtung mit den Hauptbaugruppen Mikroprozessor, Speicher, Programmspeicher, Handy-Einheit mit Telefon-Schaltkreis und PC-Schnittstelle, Sende-Empfangs-Schaltkreis sind in einer Elektronik-Baugruppe 22 zusammengefaßt und befinden sich gemeinsam mit der Stromversorgung 25, z.B. eine Batterie, innerhalb einer Gehäuseabdeckung 19, die über ein Scharnier 20 an der Gehäusegrundplatte 1 angelenkt und mittels einer Verriegelung 17 geschlossen ist.

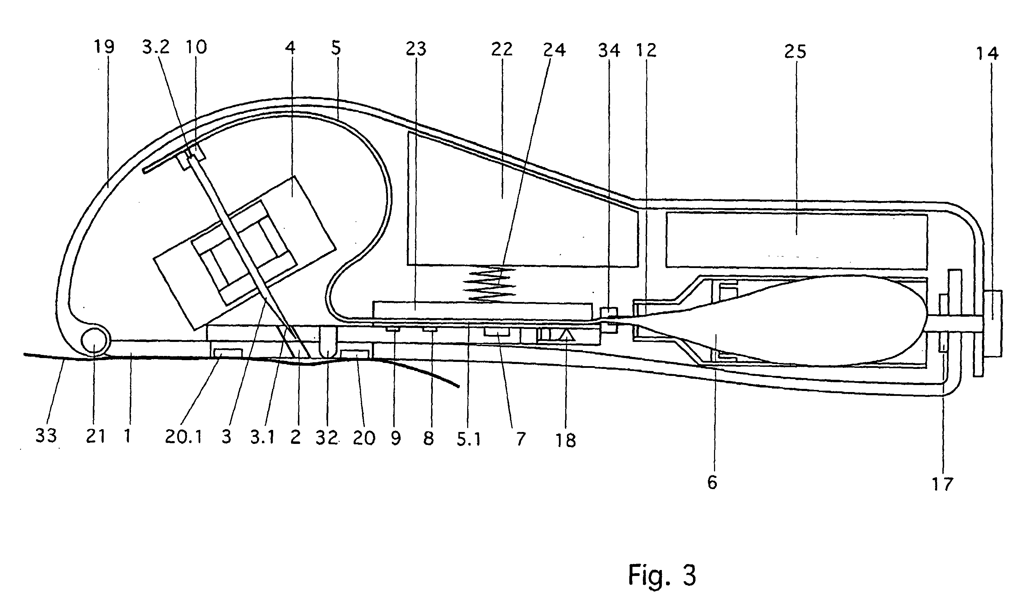

In Fig. 3 ist die Anordnung des Depots 6 beispielsweise vordergründig und parallel zur Kartusche 11 näher dargestellt. Das Depot 6 (Fig. 4) als Bestandteil des Folienverbundes 26 ist aus einem flexiblen, resistenten Material, z.B. Polytetrafluoräthylen (PTFE), das sich beim Befüllen aus der Kartusche 11 schlauchförmig aufweitet. Der Folienverbund 26 besteht aus mindestens zwei Folien, die miteinander verschweißt sind und so die erforderlichen Kanäle. Kammern und Depots bilden. Das Depot 6, ein Überströmkanal 27, das Ventil 18, die Pumpe 7, der Fluidkanal 5.1 und Fluidleitung 5 gehen so zusammen hängend ineinander über. Aus hygienischen bzw. aseptischen Gründen ist dieser Folienverbund 26 besonders vorteilhaft und als kostengünstiges Wechselelement leicht austauschbar. An den Öffnungsstellen, z.B. am Ende des Fluidkanales 5.1 und am Einlauf des Über strömkanales 27 befinden sich jeweils eine Halterung 10 für die Injektionsnadel 3 und eine Halterung 34 für die Kontaktnadel 12. In der Fig. 4.1 ist zwischen der Halterung der Kontaktnadel 12 und dem Überströmkanal 27 das weitere Ventil 35 angeordnet, das nach dem Füllvorgang des Depots 6 geschlossen wird und das Kanalsystem abdichtet. Bei dieser Ausführung des Folienverbundes 26 ist die 5 Kartusche 11 nach dem Befüllen der Depots 6 aus der Vorrichtung herausnehmbar.

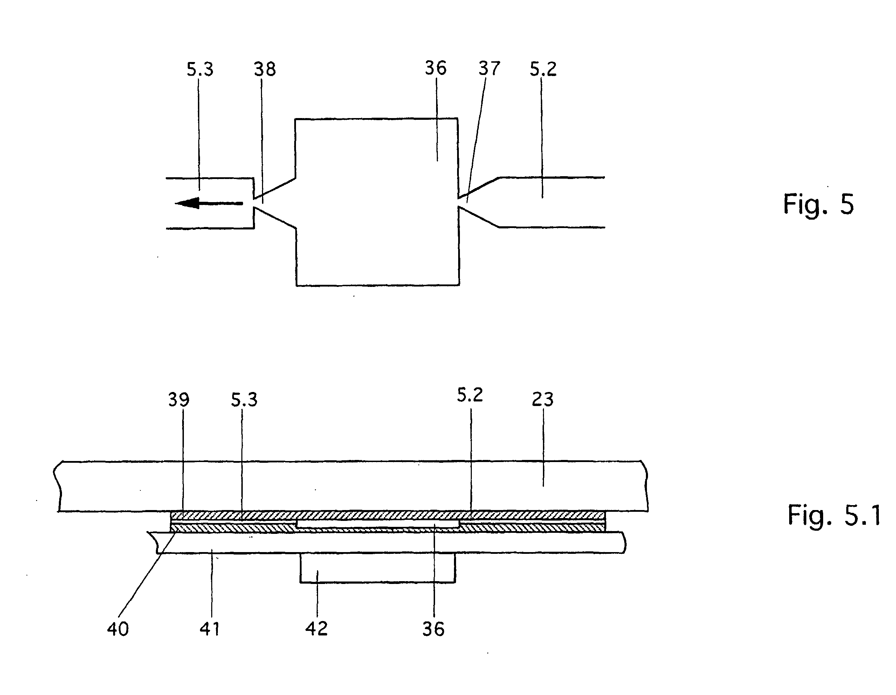

In den Fig. 5 und 5.1 ist eine Ausführung der eingesetzten Fluidpumpe 7 näher dargestellt. Diese besteht bekannterweise aus einer Pumpenkammer 36 mit einem Zulauf 5.2 und einem Ablauf 5.3. Der Ein- und Ausgang in und aus der Pumpkammer 36 sind dabei als Konfuser 37 und 38 mit jeweils trichterförmigem Querschnitt gestaltet. Die Konfuser 37, 38 sind richtungsabhängige Strömungswiderstände und bewirken einen richtungsbezogenen Fluß. Der Kanalkörper 40 mit der Pumpkammer 36 ist einerseits mit einer Abdeckfolie 39 verschweißt und trägt auf der gegenüberliegenden Seite eine Piezoscheibe 42. Zwischen der Piezoscheibe 42 und dem Kanalkörper 40 ist eine Metallmembran 41 angeordnet, die sich bei aktiven Piezoelement durchbiegt und eine Druckwelle bewirkt. Beim Schließen des Gehäusedeckels 19 legt sich eine Druckplatte 23 gegen die den Fluidkanal 5.1 abdeckende Abdeckfolie 39 und sichert so die Funktionssicherheit der Pumpe 7, da die Abdeckfolie 39 eine notwendige stabile Anlage erhält. Bei Anlegen einer Wechselspannung an die Piezoscheibe 42 wird also das im Kanalsystem befindliche Fluid verdrängt und in definierten Mengen in Richtung der Injektionsspritze 3.1 bewegt.

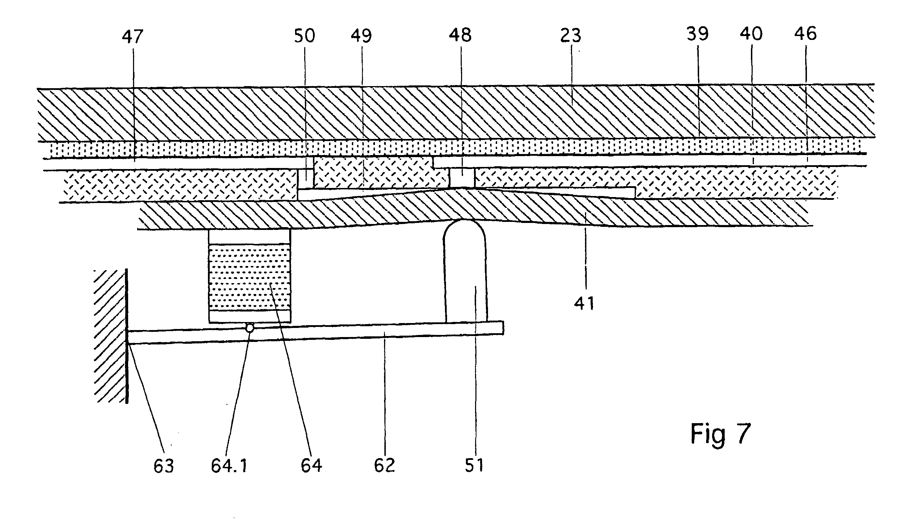

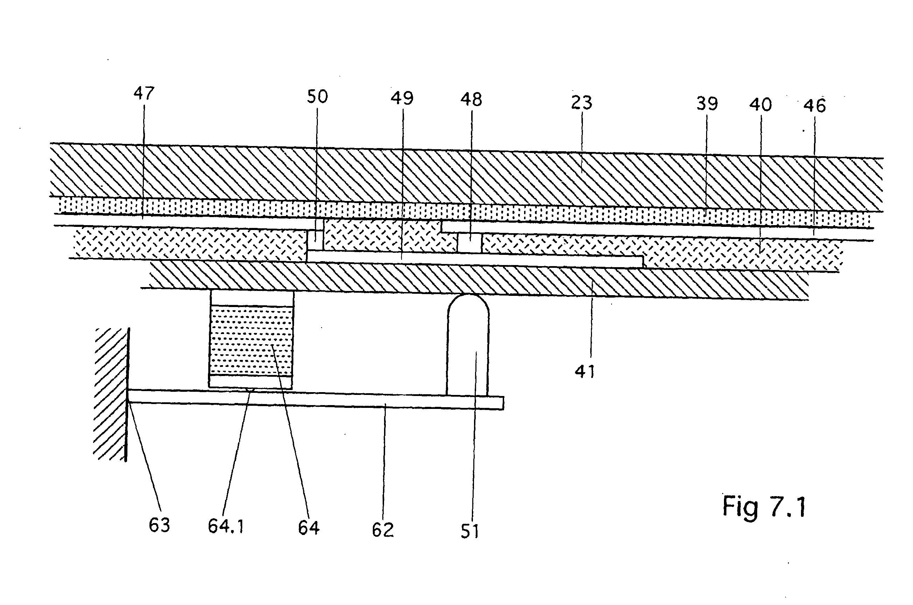

Funktionsbedingt ist in den Fluidkanal 5.1 ein Ventil 18 ( Fig. 7 und 7.1) eingesetzt. Bei Füllen der Depots 6 aus der Kartusche 11 muß das Ventil 18 beispielsweise geschlossen werden, damit das Fluid nicht bereits in und durch die Injektionsnadel 3 gelangt.

Ein weiteres Ventil 35 (Fig. 4.1) ist für den Fall zweckmäßig, falls die Kartusche 11 nach der Entleerung wieder aus der Vorrichtung entnommen werden soll. Nur beim Befüllen der Depots 6 aus der Kartusche 11 ist das Ventil 35 geöffnet. Beide Ventile 18; 35 werden über einen piezoelektrischen Aktuator 64 geöffnet oder geschlossen. Im Ruhezustand des Akluators 64 ist das Ventil 18 geschlossen (Fig.7). Eine Einlaßöffnung 48 in eine Ventilkammer 49 eines Kanalkörpers 40 wird dabei von einer die Ventilkammer 49 abdeckenden Metallmembran 41 geschlossen gehalten, indem ein an einer vorgespannten Blattfeder 62 angelenktes Druckstück 51 gegen die Metallmembran 41 drückt. Im Bereich der Einlaßöffnung 48 weist die Metallmembran 41 eine Beschichtung aus einem Dichtungsmaterial , z.B. Latex, auf, damit ein sicherer Verschluß an der Einlaßöffnung 48 erreicht ist. Zwischen der an einem Gestell 63 befestigten Blattfeder 62 und der Metallmembran 41 ist ein piezoelektrischer Aktuator 64 angeordnet, der aus einem Stapel von Piezoelementen besteht und sich bei Anlegen einer Spannung ausdehnt und gegen die Blattfeder 62 drückt und die Metallmembran 41 entlastet. Die Metallmembran 41 geht in ihre Ausgangslage (Fig. 7.1 ) zurück und die Einlaßöffnung 48 des Ventils 18 ist wieder geöffnet. Die Ventile 18 und 35 sind so in einfacher Weise und ohne besonderen Energieaufwand steuerbar.

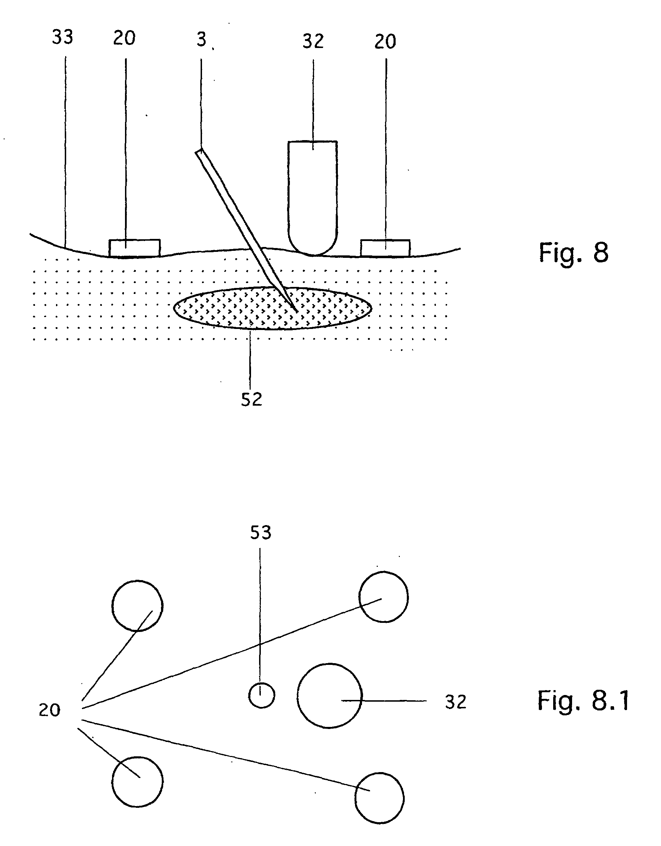

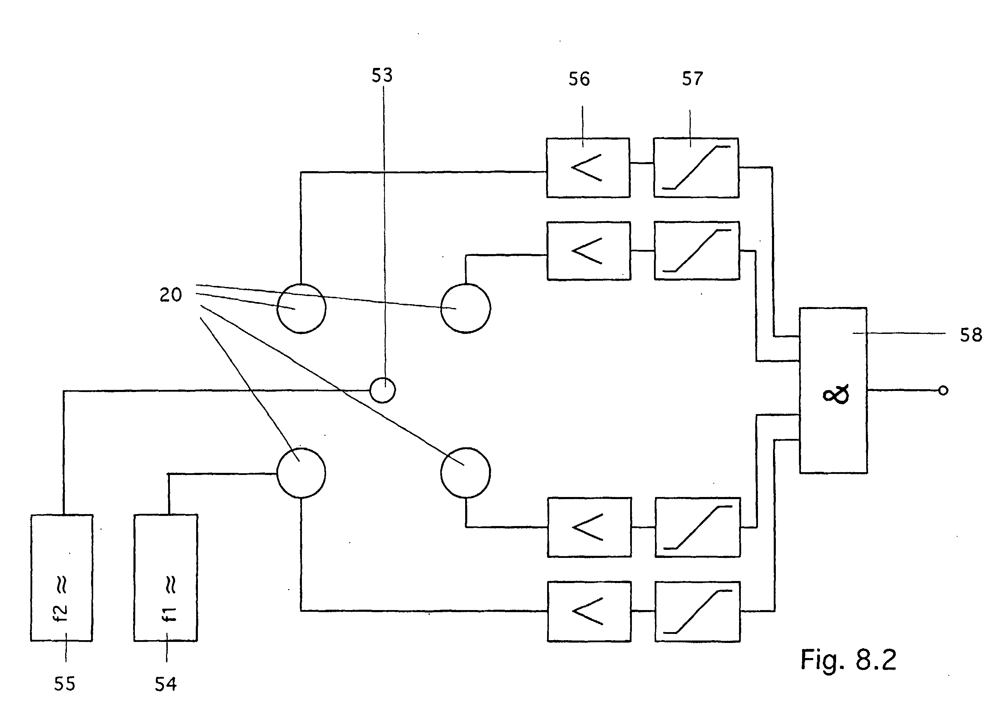

In Fig. 8 und 8.1 sind die prinzipienhafte Anordnung der Auflagesensoren 20, der Injektionsnadel 3 und des Infrarotsensors 32 zueinander dargestellt. Nur bei sicherer Auflage der Vorrichtung auf der Hautoberfläche 33 durch ein entsprechendes elektrisches Signal aller Auflagesensoren 20 wird die Vorrichtung in Betriebsbereitschaft versetzt und die Injektionsnadel 3 kann in die Haut 33 eingeführt werden. Die gespeicherte bzw. programmierte Injektionsmenge wird anschließend in die Haut gepumpt. Eine Kontrolle über die Ausführung der Injektion des Medikamentes in die Haut erfolgt über einen temperaturempfindlichen IR-Sensor 32, der nahe der Einstichstelle der Injektionsnadel 3 in der Gchäusegrundplatte 1 angeordnet ist. Die eingepumpte Medikamentenmenge 52 erzeugt einen Temperaturabfall der unter dem IR-Sensor liegenden Hautschicht, so daß dieser ein entsprechendes auswertbares Signal liefert und einen erfolgreichen Injektionsvorgang an die Informationsverarbeitungseinheit 74 meldet.

Fig. 8.2 stellt ein elektrisches Ersatzschaltbild für die Auswertung der korrekten Auflage der Sensoren 20 dar. An einer der Auflagesensoren 20 liegt eine Niedrigspannung U mit einer Frequenz f1 eines Frequenzgenerators 54 an. Gleichzeitig sind alle Auflagesensorcn 20 mit einer Auswerteschaltung 56 bis 58 verbunden, die nur dann ein Bereitschaftssignal sendet, wenn an allen Auflagesensoren 20 ein auswertbares Signal (Fig. 8) mit der Frequenz f1 registriert wird. Die einzelnen Sensoren 20 sind dazu mit einem Vorverstärker 56 und einem Begrenzer 57 verbunden, deren Signale an ein UND-Gatter 58 geführt sind.
Zur Kontrolle der Penetration der Injektionsnadel 3 wird im Ablauf zeitlich versetzt eine Wechselspannung mit der Freuquenz f2 des Frequenzgenerators 55 an die Injektionsnadel 3 gelegt und ebenfalls über die Auflagesensoren 20 und der Auswerteschaltung 56 bis 58 ausgewertet.
Wird auch bei dieser Kontrolle ein Betätigungssignal am Ausgang des UND-Gatters 58 festgestellt, ist die Bereitschaft der Vorrichtung hergestellt, und die Injektion in die Haut des Patienten kann beginnen.

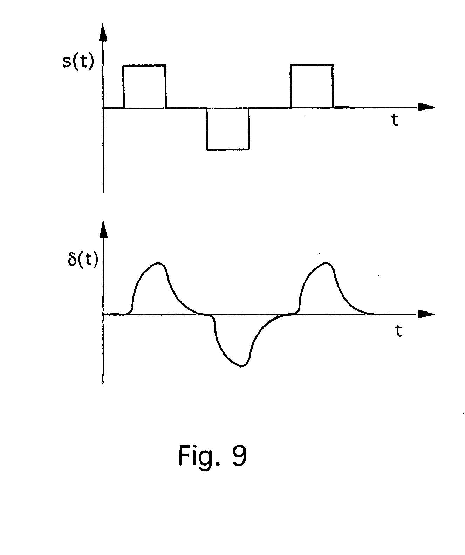

In Fig. 9 sind in einem Impulsdiagramm die auswertbaren Signale dargestellt, wie sie mit der Schaltung aus Fig. 8.2 entstehen.
Mittels der Sensorik ist die Vorrichtung in der Lage, ihre Funktionsabläufe selbst zu kontrollieren. Fehlmeldungen werden registriert, optisch oder akustisch angezeigt und über den Übertragungsmodul 70 an die externe Zentralstelle gemeldet.
Im Übertragungsmodul 70 ist darüberhinaus ein Handy-Telefon-Schaltkreis, so daß mit der Vorrichtung über ein vorhandenes Funknetz sprachlich mit der Zentralstelle kommuniziert werden kann.

## Patentansprüche

1. Vorrichtung zur Injektion eines Medikamentes dosierter Menge mit einem Behälter, einer Injektionskanüle, einer Fördereinrichtung für das Medikament und einem Übertragungsmodul, wobei die Vorrichtung am Körper eines Patienten tragbar ist, **gekennzeichnet durch** eine räumliche und funktionelle Verbindung
- eines geschlossenen Fluidiksystems (26) mit von außen am Fluidsystem wirksam angeordneter
- Dosiereinrichtung mit angeschlossenem Depot (6), und einer Befüllstation des Depots (6) aus einer Kartusche (11), sowie einer Fördereinrichtung (7) für das Therapeutikum,
- eines steuerbaren Antriebes (4) für die Penetration der an das Fluidiksystem lösbar angeschlossenen Injektionskanüle (3),
- eines Überwachungsmodules (72) mit einer Meßeinrichtung der injizierten Menge, einem Sensorsystem (9;20;20.1;32) zur Kontrolle der Auflage der Vorrichtung auf der Hautoberfläche, zur Kontrolle der Penetration der Injektionsnadel in der Haut und zur Kontrolle der Ausführung der Injektion in Verbindung mit einer Auswerteschaltung,
- einer Informationsverarbeitungseinheit (74) mit Mikroprozessor (73), Hauptspeicher und einer elektronischen Zeit-Mengen-Steuerung mit Programmspeicher und Registern für MENGE, ZEIT und Sonderfunktionen,
- eines Übertragungsmodules (70) mit einem GSM (Modem) für die wechselseitige Datenübertragung von Meßwerten , Stellgrößen, Programmänderungen und mit einem Handy-Telefon-Schaltkreis für die separate Sprachverbindung an eine externe Zentralstation.

2. Vorrichtung nach Anspruch 1, **dadurch gekennzeichnet, daß** das in sich geschlossene Fluidiksystem aus einem flexiblen Folienverbund (26) von mindestens zwei strukturierten und übereinanderliegenden fest miteinander verbundenen Folien besteht.

3. Vorrichtung nach Anspruch 2, **dadurch gekennzeichnet, daß** der in sich geschlossene Folienverbund (26) verschweißt ist und aus Polytetrafluoräthylen besteht.

4. Vorrichtung nach Anspruch 1, **dadurch gekennzeichnet, daß** die Dosiereinrichtung aus einer gesteuerten piezoelektrischen Mikropumpe (7), angeordnet an einem Fluidkanal (5.1) und zwischen einem Medikament-Depot (6) und der Injektionskanüle (3), besteht, wobei der piezoelektrischen Mikropumpe (7) ein Flußmesser für den SOLL-IST-Vergleich der Zeit-Mengen-Steuerung nachgeordnet ist .

5. Vorrichtung nach Anspruch 1 bis 4, **dadurch gekennzeichnet, daß** der Fluidkanal (5.1) mit angeschlossener Mikropumpe (7) und Flußmessung mit der sich anschließenden flexiblen Fluidleitung (5) für den Anschluß der Injektionskanüle (3) und dem Medika - menten-Depot (6) als geschlossenes Fluidiksystem ausgebildet, austauschbar ist.

6. Vorrichtung nach Anspruch 5, **dadurch gekennzeichnet, daß** das geschlossene Fluidiksystem am Einlauf eine Halterung (34) für eine Kontaktnadel (12) und am Auslauf der Fluidleitung (5) eine Halterung (10) für den Anschluß einer Injektionskanüle (3) aufweist.

7. Vorrichtung nach Anspruch 6, **dadurch gekennzeichnet, daß** die Kontakt nadel (12) beim Einlegen einer Kartusche (11) in eine elastische Verschlußkappe (13) der Kartusche (11) abdichtend eindringt.

8. Vorrichtung nach Anspruch 7, **dadurch gekennzeichnet, daß** die Verschluß kappe (13) der Kartusche (11) aus Naturkautschuk besteht.

9. Vorrichtung nach Anspruch 2, **dadurch gekennzeichnet, daß** die Flußmessung für die zu fördernde Medikamenten-Menge durch die Anordnung eines Temperatursensors (9) und eines Peltierelementes (8) realisiert ist, die in definiertem Abstand am Ausgang der Mikropumpe (7) angeordent sind.

10. Vorrichtung nach Anspruch 1 bis 5, **dadurch gekennzeichnet, daß** der Mikropumpe (7) ein Einlaßventil (18) vorgeschaltet ist und die Pumpkammer der Mikropumpe (7) und Ventilkammer des Einlaßventils (18) in einem Kanalkörper (40) strukturiert ein geformt sind.

11. Vorrichtung nach Anspruch 10, **dadurch gekennzeichnet, dass** der Mikropumpe (7) am Zulauf und am Ablauf Konfuser (37; 38) als richtungsbezogene Strömungswiderstände zugeordnet sind.

12. Vorrichtung nach Anspruch 1, **dadurch gekennzeichnet, dass** der Antrieb für die Injektionskanüle (3) ein Linearantrieb ist, der aus einem Schubverband von piezoelektrischen Aktoren besteht, an die in einer programmierten Steuerfolge eine Spannung U angelegt wird und die Injektionskanüle (3) linear im spitzem Winkel aus einer Öffnung (2) der Gehäusegrundplatte (1) führt.

13. Vorrichtung nach Anspruch 12, **dadurch gekennzeichnet, dass** der spitze Winkel für den Austritt der Injektionskanüle (3) aus der Vorrichtung 45 bis 60° beträgt.

14. Vorrichtung nach Anspruch 13, **dadurch gekennzeichnet, dass** der aus piezo-elektrischen Aktoren bestehende Schubverband aus zwei Klemmringen (43; 44) und einem zwischen den Klemmringen (43; 44) liegendem Schubring (45) besteht, wobei die Klemmringe eine mittige Bohrung (43.1; 44.1) mit Klemmflächen (43.1; 44.1) zur Durchführung der Injektionskanüle (3) aufweisen, wobei ein Klemmring fest am Chassis angeordnet ist.

15. Vorrichtung nach Anspruch 1, **dadurch gekennzeichnet, dass** zur Überwachung der Körperauflage mindestens drei Körperelektroden (20) angeordnet sind, deren Signale an eine logische Schaltung (56 bis 58) geführt sind, die bei Körperauflage ein Bereitschaftssignal an die Informationsverarbeitungseinheit (74) sendet.

16. Vorrichtung nach Anspruch 15, **dadurch gekennzeichnet, dass** an eine der Körperelektroden (20) eine Wechselspannung mit der Frequenz f1 geführt ist, die über die weiteren Körperelektroden (20) übertragen und als Einzelsignale über Vor verstärker (56), Begrenzer (57) an eine UND-Verknüpfung (58) geführt sind.

17. Vorrichtung nach Anspruch 16, **dadurch gekennzeichnet, dass** an die Injektionskanüle (3) eine Wechselspannung mit der Frequenz f2 geführt ist, die zeitlich versetzt über alle Körperelektroden (20) übertragen und als Einzelsignale über Vorverstärker (56), Begrenzer (57) an die UND-Verknüpfung (58) geführt sind.

18. Vorrichtung nach Anspruch 1, **dadurch gekennzeichnet, dass** die in der Informationsverarbeitungsanlage gespeicherten Programme und die Inhalte der Register MENGE, ZEIT und Sonderfunktionen über das GSM des Übertragungsmoduls (70) von einer externen Zentralstelle änderbar sind , sowie die Informationsverarbeitungsanlage Eingänge für die Sensorsignale aufweist.

19. Vorrichtung nach Anspruch 15, **dadurch gekennzeichnet, dass** ein Auflagesensor (20) mit einem Frequenzgenerator mit der Frequenz f1 in Verbindung steht, die von allen Auflagesensoren an eine Auswerteschaltung übertragen wird und über Vorverstärker (56), Begrenzer (57) und UND-Gatter ein Ausgangssignal bildet.

20. Vorrichtung nach Anspruch 19, **dadurch gekennzeichnet, daß** die Injektionskanüle (3) mit einem Frequenzgenerator mit der Frequenz f2 in Verbindung steht, die von allen Auflagesensoren (20) empfangen wird und diese über Vorverstärker (56) und Begrenzer (57) als Ausgangssignale an die Auswerteschaltung weiterleitet.

## Claims

1. Device for the injection of a medicament of a dosed amount with a container, an injection cannula, a delivery means for the medicament and a transmission module, the device being portable on the body of a patient, **characterized by** a spatial and functional combination
- of a closed fluidic system (26) with, arranged so as to be effective from the outside on the fluid system,
- a dosing means with a connected depot (6), and a filling station of the depot (6) comprising a cartridge (11), and also a delivery means (7) for the therapeutic agent,
- and a controllable drive (4) for the penetration of the injection cannula (3) detachably connected to the fluidic system,
- of a monitoring module (72) with a means for measuring the injected amount, a sensor system (9; 20; 20.1; 32) for checking the contact of the device on the surface of the skin, for checking the penetration of the injection needle in the skin and for checking the performance of the injection in conjunction with an evaluation circuit,
- of an information processing unit (74) with a microprocessor (73), main memory and an electronic time-amount control with a program memory and registers for AMOUNT, TIME and special functions,
- of a transmission module (70) with a GSM (modem) for the reciprocal data transmission of measured values, manipulated variables, program changes and with a mobile telephone circuit for the separate voice connection to an external central station.

2. Device according to Claim 1, **characterized in that** the self-contained fluidic system comprises a flexible film laminate (26) of at least two films that are structured and, one lying on top of the other, are firmly connected to each other.

3. Device according to Claim 2, **characterized in that** the self-contained film laminate (26) is welded and consists of polytetrafluoroethylene.

4. Device according to Claim 1, **characterized in that** the dosing means comprises a controlled piezoelectric micropump (7), arranged on a fluid channel (5.1) and between a medicament depot (6) and the injection cannula (3), with a flow meter for the SET-ACTUAL comparison of the time-amount control being arranged downstream of the piezoelectric micropump (7).

5. Device according to Claims 1 to 4, **characterized in that** the fluid channel (5.1) with the connected micropump (7) and flow measurement with the adjoining flexible fluid line (5) for the connection of the injection cannula (3) and the medicament depot (6), formed as a closed fluidic system, is exchangeable.

6. Device according to Claim 5, **characterized in that** the closed fluidic system has at the inlet a holder (34) for a contact needle (12) and at the outlet of the fluid line (5) a holder (10) for the connection of an injection cannula (3).

7. Device according to Claim 6, **characterized in that**, when a cartridge (11) is placed in, the contact needle (12) penetrates in a sealing manner into a flexible closure cap (13) of the cartridge (11).

8. Device according to Claim 7, **characterized in that** the closure cap (13) of the cartridge (11) consists of natural rubber.

9. Device according to Claim 2, **characterized in that** the flow measurement for the amount of medicament to be delivered is realized by the arrangement of a temperature sensor (9) and a Peltier element (8), which are arranged with a defined spacing at the output of the micropump (7).

10. Device according to Claims 1 to 5, **characterized in that** an inlet valve (18) is arranged upstream of the micropump (7), and the pumping chamber of the micropump (7) and the valve chamber of the inlet valve (18) are formed in a structured manner in a channel body (40).

11. Device according to Claim 10, **characterized in that** confusers (37; 38) are assigned to the micropump (7) as directionally related flow resistances at the inlet and at the outlet.

12. Device according to Claim 1, **characterized in that** the drive for the injection cannula (3) is a linear drive, which comprises a pushing unit of piezoelectric actuators, to which a voltage U is applied in a programmed control sequence and the injection cannula (3) leads linearly at an acute angle out of an opening (2) in the housing base plate (1).

13. Device according to Claim 12, **characterized in that** the acute angle for the exiting of the injection cannula (3) from the device is 45 to 60°.

14. Device according to Claim 13, **characterized in that** the pushing unit comprising piezoelectric actuators comprises two clamping rings (43; 44) and a pushing ring (45) lying between the clamping rings (43; 44), the clamping rings having a central bore (43.1; 44.1) with clamping faces (43.1; 44.1) for leading the injection cannula (3) through, one clamping ring being arranged fixedly on the chassis.

15. Device according to Claim 1, **characterized in that** at least three body electrodes (20) are arranged for monitoring the contact on the body, the signals of which are led to a logic circuit (56 to 58), which sends a readiness signal to the information processing unit (74) when there is body contact.

16. Device according to Claim 15, **characterized in that** an a.c. voltage with the frequency f1 is passed to one of the body electrodes (20), transmitted via the further body electrodes (20) and passed as individual signals to an AND operation (58) via preamplifiers (56) and limiters (57).

17. Device according to Claim 16, **characterized in that** an a.c. voltage with the frequency. f2 is passed to the injection cannula (3), transmitted at time intervals via all the body electrodes (20) and passed as individual signals to the AND operation (58) via preamplifiers (56) and limiters (57).

18. Device according to Claim 1, **characterized in that** the programs stored in the information processing system and the contents of the registers AMOUNT, TIME and special functions can be changed from an external central location via the GSM of the transmission module (70), and the information processing system has inputs for the sensor signals.

19. Device according to Claim 15, **characterized in that** a contact sensor (20) is in connection with a frequency generator with the frequency f1, which is transmitted from all the contact sensors to an evaluation circuit and forms an output signal via preamplifiers (56), limiters (57) and AND gates.

20. Device according to Claim 19, **characterized in that** the injection cannula (3) is in connection with a frequency generator with the frequency f2, which is received by all the contact sensors (20) and passes it on as output signals to the evaluation circuit via preamplifiers (56) and limiters (57).

## Revendications

1. Appareil pour injecter une quantité dosée d'un médicament comprenant un récipient, une canule d'injection, un dispositif de transport pour le médicament et un module de transmission, l'appareil pouvant être porté sur le corps d'un patient, **caractérisé par** une liaison spatiale et fonctionnelle
- d'un système fluidique fermé (26) comprenant un dispositif de dosage disposé de manière à agir à l'extérieur du système fluidique auquel est raccordé un dépôt (6) et une station de remplissage du dépôt (6) à partir d'une cartouche (1) ainsi qu'un dispositif de transport (7) pour l'agent thérapeutique,
- d'un entraînement commandable (4) pour la pénétration de la canule d'injection raccordée de manière amovible au système fluidique,
- d'un module de surveillance (72) comportant un dispositif de mesure de la quantité injectée, un ensemble de capteurs (9 ; 20 ; 20.1 ; 32) pour contrôler l'application de l'appareil sur la surface de la peau, pour contrôler la pénétration de l'aiguille d'injection dans la peau et pour contrôler l'exécution de l'injection en combinaison avec un circuit d'analyse,
- d'un module de traitement des informations (74) comportant un microprocesseur (73), une mémoire principale et une commande électrique de la quantité dans le temps avec une mémoire de programmes et des registres pour la valeur QUANTITÉ.TEMPS et des fonctions spéciales,
- d'un module de transmission (70) comportant un GSM (modem) pour la transmission réciproque des valeurs mesurées, grandeurs de commande, modifications de programmes et comportant un circuit de commande de téléphone portable pour la liaison vocale séparée avec une station centrale externe.

2. Appareil selon la revendication 1, **caractérisé en ce que** le système fluidique fermé en lui-même se compose d'une combinaison flexible de films (26) constituée d'au moins deux films structurés reliés à demeure l'un à l'autre de manière superposée.

3. Appareil selon la revendication 2, **caractérisé en ce que** la combinaison de films (26) fermée en elle-même est soudée et se compose de polytétrafluoréthylène.

4. Appareil selon la revendication 1, **caractérisé en ce que** le dispositif de dosage se compose d'une micropompe à commande piézoélectrique (7) montée sur un canal à fluide (5.1) et entre un dépôt de médicament (6) et la canule d'injection (3), un débitmètre destiné à la comparaison CONSIGNE/RÉEL de la commande de la quantité dans le temps étant branché en aval de la micropompe piézoélectrique (7).

5. Appareil selon les revendications 1 à 4, **caractérisé en ce que** le canal à fluide (5.1) auquel sont raccordées la micropompe (7) et la mesure du débit avec la conduite à fluide flexible (5) qui est raccordée est réalisé sous la forme d'un système fluidique fermé pour le raccordement de la canule d'injection (3) et du dépôt de médicament (6) et peut être remplacé.

6. Appareil selon la revendication 5, **caractérisé en ce que** le système fluidique fermé présente à l'entrée un support (34) pour une aiguille de contact (12) et à la sortie de la conduite à fluide (5) un support (10) pour le raccordement d'une canule d'injection (3).

7. Appareil selon la revendication 6, **caractérisé en ce que** l'aiguille de contact (12), lors de l'insertion d'une cartouche (11), pénètre dans un capuchon de fermeture élastique (13) de la cartouche (11) en établissant l'étanchéité.

8. Appareil selon la revendication 7, **caractérisé en ce que** le capuchon de fermeture (13) de la cartouche (11) est en caoutchouc naturel.

9. Appareil selon la revendication 2, **caractérisé en ce que** la mesure du débit de la quantité de médicament à transporter est réalisée par l'arrangement d'un capteur de température (9) et d'un élément de Peltier (8) qui sont disposés à un écart donné à la sortie de la micropompe (7).

10. Appareil selon les revendications 1 à 5, **caractérisé en ce qu'**une vanne d'entrée (18) est branchée en amont de la micropompe (7) et la chambre de pompe de la micropompe (7) et la chambre de vanne de la vanne d'entrée (18) sont formées de manière structurée dans un corps de canal (40).

11. Appareil selon la revendication 10, **caractérisé en ce que** des embrouilleurs (37 ; 38) faisant office de résistances à l'écoulement directionnelles sont disposés à l'entrée et à la sortie de la micropompe (7).

12. Appareil selon la revendication 1, **caractérisé en ce que** l'entraînement de la canule d'injection (3) est un entraînement linéaire qui se compose d'un train coulissant d'actionneurs piézoélectriques auxquels est appliquée une tension U dans un ordre de commande programmé et qui guident la canule d'injection (3) de manière linéaire selon un angle aigu à partir d'une ouverture (2) de la base du boîtier (1).

13. Appareil selon la revendication 12, **caractérisé en ce que** l'angle aigu de sortie de la canule d'injection (3) de l'appareil est compris entre 45 et 60°.

14. Appareil selon la revendication 13, **caractérisé en ce que** le train coulissant constitué d'actionneurs piézoélectriques se compose de deux bagues de serrage (43 ; 44) et d'une bague coulissante (45) qui se trouve entre les bagues de serrage (43 ; 44), les bagues de serrage présentant ici un orifice central (43.1 ; 44.1) avec des faces de serrage (43.1 ; 44.1) pour faire passer la canule d'injection (3), une bague de serrage étant fixée à demeure au châssis.

15. Appareil selon la revendication 1, **caractérisé en ce qu'**au moins trois électrodes de corps (20) sont placées pour surveiller l'application sur le corps, électrodes de corps dont les signaux sont acheminés à un circuit logique (56 à 58) qui envoie un signal de disponibilité au module de traitement des informations (74) en cas d'application sur le corps.

16. Appareil selon la revendication 15, **caractérisé en ce qu'**à l'une des électrodes de corps (20) est acheminée une tension alternative de fréquence f1, laquelle est transmise par les autres électrodes de corps (20) et acheminée sous la forme de signaux individuels à une porte ET (58) par le biais d'un préamplificateur (56), limiteur (57).

17. Appareil selon la revendication 16, **caractérisé en ce qu'**à la canule d'injection (3) est acheminée une tension alternative de fréquence f2, laquelle est transmise avec un décalage dans le temps sur toutes les électrodes de corps (20) et acheminée sous la forme de signaux individuels à une porte ET (58) par le biais d'un préamplificateur (56), limiteur (57).

18. Appareil selon la revendication 1, **caractérisé en ce que** les programmes enregistrés dans l'équipement de traitement des informations et le contenu des registres QUANTITÉ.TEMPS ainsi que des fonctions spéciales peuvent être modifiés par le biais du GSM du module de transmission (70) depuis un poste central externe et l'équipement de traitement des informations présente des entrées pour les signaux des capteurs.

19. Appareil selon la revendication 15, **caractérisé en ce qu'**un capteur d'application (20) est en liaison avec un générateur de fréquence ayant une fréquence f1, laquelle est transmise par tous les capteurs d'application à un circuit d'analyse et, par le biais du préamplificateur (56), du limiteur (57) et de la porte ET, forme un signal de sortie.

20. Appareil selon la revendication 19, **caractérisé en ce que** la canule d'injection (3) est en liaison avec un générateur de fréquence ayant une fréquence f2, laquelle est reçue par tous les capteurs d'application (20) qui la transmettent au circuit d'analyse sous la forme de signaux de sortie par le biais du préamplificateur (56) et du limiteur (57).
